(19) Europäisches Patentamt
European Patent Office
Office européen des brevets

(11) Publication number: **0 350 012**
**A2**

(12) **EUROPEAN PATENT APPLICATION**

(21) Application number: 89112284.8

(22) Date of filing: 05.07.89

(51) Int. Cl.4: **A61K 31/445**

(30) Priority: 08.07.88 JP 171376/88

(43) Date of publication of application:
10.01.90 Bulletin 90/02

(84) Designated Contracting States:
BE DE ES FR GB IT NL SE

(71) Applicant: MEIJI SEIKA KAISHA LTD.
4-16 Kyobashi 2-chome
Chuo-ku Tokyo 104(JP)

Applicant: National Institute of Health
No. 10-35, Kamiosaki 2-chome
Shinagawa-ku Tokyo(JP)

(72) Inventor: Yamamoto, Haruo c/o
Pharmaceutical Research Lab.
Meiji Seika Kaisha Ltd. 760, Morooka-machi
Kohoku-ku Yokohama-shi Kanagawa-ken(JP)
Inventor: Tsuruoka, Takashi c/o
Pharmaceutical Research Lab.
Meiji Seika Kaisha Ltd. 760, Morooka-machi
Kohoku-ku Yokohama-shi Kanagawa-ken(JP)
Inventor: Yoshida, Kiyoshi c/o Pharmaceutical
Research Lab.
Meiji Seika Kaisha Ltd. 760, Morooka-machi
Kohoku-ku Yokohama-shi Kanagawa-ken(JP)
Inventor: Inouye, Shigeharu c/o
Pharmaceutical Research Lab.
Meiji Seika Kaisha Ltd. 760, Morooka-machi
Kohoku-ku Yokohama-shi Kanagawa-ken(JP)
Inventor: Shimizu, Hiroyuki c/o
Pharmaceutical Research Lab.
Meiji Seika Kaisha Ltd. 760, Morooka-machi
Kohoku-ku Yokohama-shi Kanagawa-ken(JP)
Inventor: Kitamura, Takashi c/o National
Institute of Health
10-35, Kamiosaki 2-chome
Shinagawa-ku Tokyo-to(JP)

(74) Representative: Patentanwälte Deufel- Schön-
Hertel- Lewald- Otto
Isartorplatz 6
D-8000 München 2(DE)

(54) **Antiviral composition.**

(57) N-Substituted-1-deoxynojirimycin derivatives such as N-(3-phenylpropyl)-1-deoxynojirimycin can effectively inhibit the formation of giant cells and are useful for therapy and prophylaxis of AIDS.

EP 0 350 012 A2

# ANTIVIRAL COMPOSITION

## BACKGROUND OF THE INVENTION

### Field of the Invention

The present invention relates to an antiviral composition comprising an N-substituted-1-deoxynojirimycin derivative as an effective ingredient, and more particularly to an antiviral composition for prophylaxis and therapy of patients infected with retro viruses including AIDS virus.

### Description of Prior Art

Acquired immunodeficiency syndrome (AIDS) is a disease quite new to human. In recent years, AIDS has prevailed on a worldwide scale and become a serious social problem.

AIDS is characterized by serious reduction of immune functions and appearance of nervous conditions due to infection with human immunodeficiency virus (HIV) classified into retro virus and is a fatal disease complicated with opportunistic infection or malignant tumor such as Kaposi's sarcoma, etc. The opportunistic infection is caused by pathogenic microorganisms, for example including Pneumocytis carinii, protozoa, fungi, viruses, bacteria, etc. Further, Kaposi's sarcoma, non-Hodgkin's disease, primary lymphoma, etc. are known as malignant tumor complications.

Drugs for treating AIDS have now been actively investigated around the world. With respect to, for example, antiviral azide thymidine (AZT), the effect of increasing life span of the patient with AIDS has been clinically established.

However, the known antiviral agent described above is causing side effects such as myeloid disturbance, anemia or headache, nervous symptoms such as spasm, etc.

## SUMMARY OF THE INVENTION

An object of the present invention is to provide antiviral compositions, especially anti-retroviral compositions,which are free of the problems involved in the known antiviral agent.

The present inventors have found that N-substituted-1-deoxynojirimycin derivatives which are in part known significantly prevent human T lymphocytes infected with viruses, especially with AIDS virus (HIV), from death due to formation of giant cells.

The present invention is directed to an antiviral composition, especially an antiviral composition for prophylaxis and thereby of disease with retro viruses including AIDS virus, comprising as an effective ingredient a N-substituted-1-deoxynojirimycin derivative represented by general formula (1):

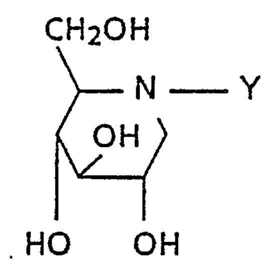

wherein Y represents

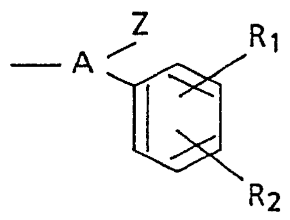

(wherein A represents a straight or branched chain hydrocarbon group having 3 to 5 carbon atoms which may or may not contain a double bond, $R_1$ and $R_2$, which may be the same or different, each represents a hydrogen atom, a lower alkyl group, a lower alkoxy group, a carboxyl group, a carbamoyl group, and alkoxycarbonyl group, a dialkylamino group or a halogen atom; Z represents a hydrogen atom, a lower alkyl group, a phenyl group or a group shown by:

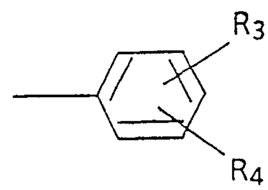

wherein $R_3$ and $R_4$, which may be the same or different, each represents a lower alkyl group, a lower alkoxy group, a carboxyl group, a carbamoyl group, an alkoxycarbonyl group, a dialkylamino group or a halogen atom) or $-(CH_2)_nCF_3$ (n represents an integer of 2 - 5), the alkyl moieties of the above mentioned groups preferably having 1 to 5 and more preferably 1 to 3 carbon atoms.

## DETAILED DESCRIPTION OF PREFERRED EMBODIMENTS

The N-substituted-1-deoxynojirimycin derivatives represented by general formula (1) which are the effective ingredients of the present invention are disclosed in Japanese Unexamined Patent Publication (Kokai) Nos. 9051/1980, 47655/1980, etc. as compounds having an effect of preventing an increase of blood sugar and synthesized in a manner similar to the methods disclosed therein.

1-Deoxynojirimycin which is a material for synthesizing the compounds of the present invention is a compound obtained by reducing nojirimycin (5-amino-5-deoxy-D-glucopyranose) [cf. Japanese Examined Patent Publication (Kokoku) No. 760/1968] (Tetrahedron, 24, 2125 - 2144, 1968).

Examples of the N-substituted-1-deoxynojirimycin derivatives which are the active ingredients of the present compositions are the following compounds, however the invention is not limited to these examples:

N-(3-phenylpropyl)-1-deoxynojirimycin, N-(4-phenylbutyl)-1-deoxynojirimycin, N-(3-phenylbutyl)-1-deoxynojirimycin, N-(3-phenylpropyl)-1-deoxynojirimycin, N-(5-phenylpentyl)-1-deoxynojirimycin, N-(3-phenyl-2-propenyl)-1-deoxynojirimycin, N-(4-phenyl-3-butenyl)-1- deoxynojirimycin, N-(3-methyl-3-phenyl-2-propenyl)-1-deoxynojirimycin, N-(3-m-methylphenyl-2-propenyl)-1-deoxynojirimycin, N-(3-p-methylphenyl-2-propenyl)-1-deoxynojirimycin, N-(3-O-chlorophenyl-2-propenyl)-1-deoxynojirimycin, N-(3-p-methylphenyl-3-methyl-2-propenyl)-1-deoxynojirimycin, N-(3-p-bromophenyl-2-propenyl)-1-deoxynjirimycin, N-(3-m-methoxyphenyl-2-propenyl)-1-deoxynojirimycin, N-(3-p-cyanophenyl-2-propenyl)-1-deoxynojirimycin, N-(3-p-carboxyphenyl-2-propenyl)-1-deoxynojirimycin, N-(3-p-chlorophenyl-3-methyl-2-propenyl) 1-deoxynojirimycin, N-(3-m-methoxyphenyl-3-methyl-2-propenyl)-1-deoxynojirimycin, N-(4-p-chlorophenyl-3-butenyl)-1-deoxynojirimycin, N-(4-m-fluorophenyl-4-methyl-3-butenyl)-1-deoxynojirimycin, N-(4-p-demethylaminophenyl-4-methyl-3-butenyl)-1-deoxynojirimycin, N-(3-p-dimethylaminophenyl-2-propenyl)-1-deoxynojirimycin, N-(3,3-diphenyl-2-propenyl)-1-deoxynojirimycin, N-(4,4-diphenyl-3-butenyl)-1-deoxynojirimycin, N-(3,3-diphenylpropyl)-1-deoxynojirimycin, N-(3-p-methoxyphenyl-3-phenyl-2-propenyl)-1-deoxynojirimycin, N-(4-p-chlorophenylbutyl)-1-deoxynojirimycin, N-(3-O-fluorophenyl-2-propenyl)-1-deoxynojirimycin, N-(3-m-fluorophenyl-2-propenyl)-1-deoxynojirimycin, N-(3-p-fluorophenyl-2-propenyl)-1-deoxynojirimycin, N-(4,4,4-trifluorobutyl)-1-deoxynojirimycin, N-(5,5,5-trifluoropentyl)-1-deoxynojirimycin, N-(6,6,6-trifluorohexyl)-1-deoxynojirimycin, etc.

It has been clarified that acquired immunodeficiency syndrome (AIDS) is mainly caused by infection of

DC4 antigen-positive humanlymphocytes with human immunodeficiency virus (HIV). Its major conditions are serious reduction in immune function, whereby opportunistic infection, malignant tumor and nervous conditions appear.

The N-substituted-1-deoxynojirimycin derivative of the present invention is a drug which shows a high therapeutic efficient for safety and may be used as a single drug. It is also effectively used in combination with AZT or other anti-AIDS drugs, for example, 2,3-dideoxycytidine, 2,3-dideoxyadenine, interferon, interleukin-2, etc.

The route for administration of the compounds of the present invention for therapy and prophylaxis is mainly per os but also a parenteral administration including intravenous, subcutaneous, intracutaneous, and intramuscular administration or an administration in form of suppositories is possible.

The daily dosage is 100 to 1500 mg which can be given once a day in a single dose or by portions a day.

However, an accurate dose may be determined depending upon age, body weight and conditions of the patient and route and frequency of administration.

Examples of medical preparations for administration include capsules, tablets, granules, granulates, powders, etc. These preparations may be formulated with starch, lactose, mannitol, ethyl cellulose, sodium carboxymethyl cellulose, etc. as carriers, and magnesium stearate or calcium stearate as lubricants. Gelatin, gum arabic, cellulose esters, polyvinyl pyrrolidone, etc. may be uses as binders.

For the preparation of compositions for parenteral administration a germ-free aqueous solution or emulsion may be used. Examples of bases for the non-aqueous solution or emulsion include propylene glycol, polyetylene glycol, glycerine, olive oil, corn oil, ethyl oleate, etc.

The anti-HIV therapeutic agent of the present invention comprises the N-substituted-1-deoxynojirimycin derivative as the effective ingredient. The compound has a mechanism different from AZT. In order to demonstrate utility of the compounds of the present invention as therapeutic agents against acquired immunodeficiency syndrome (AIDS), anti-HIV active values of the compounds of the present invention in vitro are shown below.

Example 1

Giant cell formation inhibiting effect of N-(3-phenylpropyl)-1-deoxynojirimycin:

After a test drug was dissolved in methanol medium for tissue culture was added to the solution to make a definite concentration. The concentration of methanol was finally adjusted to less than 2 %. HIV persistent injection Molt-4 cells (Molt-4/HIV cells) were precultured for 24 hours in the presence of the compound. The precultured Molt-4/HIV cells were mixed with HIV-non-infected Molt-4 cells, whereby the compound was prepared to have the same concentration as that of the preculture. The mixture was then cultured. Giant cell formation was compared between chemically intact control cells and the group to which the compound has been added 5 and 20 hours after mixing the culture to evaluate the effect of the chemical. The evaluation was made based on the method of Yamamoto et al. (Virology, 164, 542 - 546, 1988). The evaluation effect is shown in Table 1 in terms of fusion index. At the same time of 20 hours after mixing the culture the effect of inhibiting the formation of giant cells as potent as about 90 % was noted in the group to which the compound had been added. Similarly, microscopic observation revealed that the group to which the compound hat been added clearly inhibited the formation of giant cells as compared to the control cells.

Table 1. Effect of mixed culture of Molt-4 cells and Molt-4/HIV cells on giant cell formation (Fusion index)

| Concentration (μg/ml) | Time for Mixed Culture (hours) | |
|---|---|---|
| | 5 | 20 |
| 200 | 0.23 (57.4) | 0.18 (90.5) |
| 100 | 0 (100) | 0.05 (97.4) |
| 50 | 0.19 (64.8) | 0.13 (93.2) |
| 25 | -0.07 (100) | 0.18 (90.5) |
| 12.5 | 0.11 (79.6) | 0.34 (82.1) |
| 0 | 0.54 | 1.90 |

$$\text{Fusion index} = \frac{\text{Count of live cells in a well cultured without mixing}}{\text{Count of live cells in a well tested}} - 1$$

( ): Giant cell formation inhibitory rate (%)

Fusion index was calculated based on the method of Yamamoto et al. (Vorology, 164, 542 - 546, 1988). When the giant cell formation inhibitory rates exceeds 100 % and are less than 0 % they are expressed as 100 and 0, respectively.

Example 2

Giant cell formation inhibiting effect of N-(3-phenyl-2-propenyl)-1-deoxynojirimycin:

The giant cell formation inhibiting effect of this compound was examined in a manner similar to Example 1. The results are shown in Table 2. As noted in Table 2, the compound exerted the giant cell formation inhibiting effect as potent as 90 to 100 %.

Table 2.

| Effect of mixed culture of Molt-4 cells and Molt-4/HIV cells on giant cell formation (Fusion index) | | |
|---|---|---|
| Concentration (μg/ml) | Time for Mixed Culture (hours) | |
| | 5 | 20 |
| 200 | -0.08(100) | 0.10(94.7) |
| 100 | -0.10(100) | 0.10(94.7) |
| 50 | 0.06(88.9) | -0.07(100) |
| 25 | 0.17(68.5) | -0.08(100) |
| 12.5 | 0.14(74.1) | -0.03(100) |
| 0 | 0.54 | 1.90 |

Example 3

Giant cell formation inhibiting effect of N-(3,3-diphenyl-propyl)-1-deoxynojirimycin:

The giant cell formation inhibiting effect of this compound was examined in a manner similar to Example 1. The results are shown in Table 3.

Table 3.

| Effect of mixed culture of Molt-4 cells and Molt-4/HIV cells on giant cell formation (Fusion index) | | |
|---|---|---|
| Concentration (μg/ml) | Time for Mixed Culture (hours) | |
| | 5 | 20 |
| 50 | 0.32(40.7) | 0.62(67.4) |
| 25 | 0.50(7.4) | 0.89(53.2) |
| 12.5 | 0.26(51.9) | 1.37(27.9) |
| 6.25 | 0.54(0) | 2.16(0) |
| 3.13 | 0.26(51.9) | 1.50(21.1) |
| 0 | 0.54 | 1.90 |

Example 4

Giant cell formation inhibiting effect of N-[3-(3-fluorophenyl) 2-propenyl]-1-deoxynojirimycin:

The giant cell formation inhibiting effect of this compound was examined in a manner similar to

Example 1. The results are shown in Table 4.

Table 4.

| Effect of mixed culture of Molt-4 cells and Molt-4/HIV cells on giant cell formation (Fusion index) | |
|---|---|
| Concentration (μg/ml) | Time for Mixed Culture (hours) |
| | 20 |
| 40 | 0.01(99) |
| 20 | 0.32(83) |
| 10 | 0.52(73) |
| 5 | 1.04(46) |
| 2.5 | 1.38(28) |
| 0 | 1.92 |
| ( ): Giant cell formation inhibitory rate (%) | |

Using the compound 50 % of inhibitory concentration ($IC_{50}$ value) of the giant cell formation inhibition calculated from Table 4 is 5 to 10 μg/ml.

The $IC_{50}$ values of the compounds of Examples 1 and 2 are in ranges of 5 to 10 μg/ml and 2.5 to 5 μg/ml, respectively.

Hereafter, anti-HIV effect of each of the compounds of Examples 5 through 16 is shown in Table 5 in terms of 50 % inhibitory concentration ($IC_{50}$ value) of the giant cell formation inhibition as indicated in Example 4.

Table 5

| Example | Compound | 50% Inhibitory Concentration ($IC_{50}$)μg/ml |
|---|---|---|
| 5 | N-(4-Phenylbutyl)-1-deoxynojirimycin | 5-10 |
| 6 | N-[3-(3-Chlorophenyl)-2-propenyl]-1-deoxynojirimycin | 50 |
| 7 | N-[3-(2-Chlorophenyl)-2-propenyl]-1-deoxynojirimycin | 60 |
| 8 | N-[4-(4-Chlorophenyl)-3-butenyl]-1-deoxynojirimycin | 70 |
| 9 | N-[3,3-Di(4-fluorophenyl)-2-propenyl]-1-deoxynojirimycin | >100 |
| 10 | N-[3-(4-Methylphenyl)-propyl]-1-deoxynojirimycin | 5-10 |
| 11 | N-[3-(4-Fluorophenyl)-propyl]-1-deoxynojirimycin | 5-10 |
| 12 | N-[3(4-Fuluorophenyl)-2-propenyl]-1-deoxynojirimycin | 10 |
| 13 | N-[3-(2-Fluorophenyl)-2-propenyl]-1-deoxynojirimycin | 2.5-5 |
| 14 | N-[4-(2-Fluorophenyl)-3-butenyl]-1-deoxynojirimycin | 5-10 |
| 15 | N-(4,4,4-Trifluorobutyl)-1-deoxynojirimycin | 5-10 |
| 16 | N-(5,5,5-Trifluoropentyl)-1-deoxynojirimycin | 2.5-5 |
| Control | N-(2-Phenoxyethyl)-1-deoxynojirimycin | 20-40 |

The compounds of the present invention have low toxicity. For example, in the acute toxicity test in which 4g/kg of N-(3-phenyl-2-propenyl)-1-deoxynojirimycin was administered orally to 3 mice in each group, all of the tested mice survived.

Next, examples of medical preparations of the present invention are shown below.

Example 17

| | |
|---|---|
| N-(3-Phenyl-2-propenyl)-1-deoxynojirimycin | 100 mg |
| Lactose | 250 mg |
| Potato starch | 250 mg |
| Polyvinylpyrrolidone | 20 mg |
| Magnesium stearate | 5 mg |

Lactose and potato starch are mixed and 20 % ethanolic solution of polyvinylpyrrolidone is added to the mixture to uniformly swell. The mixture is passed through a sieve having 1 mm mesh, dried at 45° C and again passed through a sieve of 1 mm mesh. The thus obtained granules are kneaded with magnesium stearate and the mixture is formed into tablets.

The antiviral agent of the present invention is a useful drug which exerts extremely superior prophylactic and therapeutic effects particularly on AIDS virus.

Hereinafter, manufacturing examples of synthesizing the N-substituted-1-deoxynojirimycin derivative which is the effective ingredient of the present invention are shown.

Manufacturing Example 1

N-(3-Phenylpropyl)-1-deoxynojirimycin

A mixture of 815 mg (5.00 mmols) of 1-deoxynojirimycin and 828 mg (6.00 mmols) of potassium carbonate was suspended in 5 ml of dimethylformamide, and 995 mg (5.00 mmols) of 1-bromo-3-phenylpropane were added to the suspension. The mixture was heated for 6 hours. After completion of the reaction, the reaction solution was diluted with 30 ml of water followed by extraction with n-butanol. After the extract was concentrated under reduced pressure, the residue was purified by silica gel column chromatography (chloroform :methanol = 10 : 1) to give 998 mg (71 %) of the product.
NMR ($CD_3OD$) δ:
1.40 (m, 2H), 2.02-2.25 (m, 2H), 2.61 (m, 2H), 3.01 (dd, 1H), 3,10 (t,1H), 3,29 (m, 1H), 3,38 (t, 1H), 3,47 (m, 1H), 3.65 (m, 1H), 3.85 (m, 2H), 7.15-7.35 (m, 5H)

Manufacturing Example 2

N-(3-Phenyl-2-propenyl)-1-deoxynojirimycin

The title compound was synthesized in a manner similar to Manufacturing Example 1 except that 1-bromo-3-phenylpropene was substituted for 1-bromo-3-phenylpropane.
NMR ($CD_3OD$) δ:
2.19 (m, 2H), 3.07 (dd, 1H), 3.16 (t, 1H), 3.28 (m, 1H), 3.14 (t, 1H), 3.50 (ddd, 1H), 3.68 (ddd, 1H), 3.95 (ABXtype, 2H), 6.35 (ddd, 1H), 6.59 (d, 1H), 7.15-7.50 (m, 5H)

Manufacturing Example 3

N-[3-(4-Fluorophenyl)propyl]-1-deoxynojirimycin

Step 1: Methyl 3-(4-fluorophenyl)-2-propenoate:

4-Fluorobenzaldehyde, 1.24 g (10.0 mmols), was dissolved in 20 ml of methylene chloride and 3.67 g (11.0 mmols) of carbomethoxymethylenetriphenylphosphorane. The mixture was stirred at room temperature for 3 hours. To the reaction mixture were added 100 ml of hexane. Solid precipitates were filtered off and filtrate was concentrated. The residue was purified by silica gel column chromatography (ethyl acetate : hexane = 1 : 4) to give 1.61 g (90 %) of colorless needles.
NMR ($CDCl_3$) δ:
4.30 (d, 2H), 6.25 (m, 1H), 6.55 (d, 1H), 6.95 (m, 2H), 7.35 (m, 2H)

Step 2: 3-(4-Fluorophenyl)-2-propen-1-ol:

Methyl-3-(4-fluorophenyl)-2-propenoate, 1.61 g (9.0 mmols), was dissolved in 50 ml of ether and under ice cooling the solution was dropwise added to a suspension of 205 mg (5.40 mmols) of aluminum lithium hydride in 3 ml of ether. After the dropwise addition the mixture was stirred for 30 minutes at room temperature. An excess of the reactant was decomposed with water and solids were filtered off. The filtrate was concentrated to give 1.33 g (97 %) of 3-(4-fluorophenyl)-2-propen-1-ol.
NMR ($CDCl_3$) δ:
4.52 (d, 2H), 6.31 (m, 1H), 7.01 (m, 2H), 7.45 (m, 2H)

Step 3: 3 (4-Fluorophenyl)propan-1-ol:

3-(4-Fluorophenyl)-2-propen-1-ol, 1.52 g (10.0 mmols), was dissolved in 50 ml of ethyl acetate and 100 mg of 10 % Pd-C was added to the solution. The mixture was catalytically reduced for 12 hours under normal pressure. After the catalyst was filtered off the filtrate was distilled to give 1.50 g of a colorless oil.
NMR ($CDCl_3$) δ:
1.91 (m, 2H), 2.70 (m, 2H), 3.68 (m, 2H), 7.16-7.36 (m, 4H)

Step 4: 3-(4-Fluorophenyl)-1-bromopropane:

3-(4-Fluorophenyl)propan-1-ol, 770 mg (5.00 mmols), and 2.49 g (10.0 mmols) of carbon tetrabromide were dissolved in 30 ml of acetonitrile and under ice cooling, 1.57 g (6.00 mmols) of triphenylphosphine was added to the solution by several portions. After stirring for 12 hours at room temperature 100 ml of hexane were added to the reaction mixture. Solid precipitates were filtered off and the filtrate was concentrated. The residue was purified by silica gel column chromatography (hexane) to give 1.06 g (91 %) of colorless oil.
NMR ($CDCl_3$) δ:
2.17 (m, 2H), 2.81 (t, 2H), 3.45 (t, 2H), 7.14 -7.36 (m, 4H)

Step 5: N-[3-(4-Fluorophenyl)propyl]-1-deoxynojirimycin

The title compound was synthesized in a manner similar to Manufacturing Example 1 except that 3-(4-fluorophenyl)-1-bromopropane was substituted for 1-bromo-3-phenylpropane.
NMR ($CD_3OD$) δ:
1.38 (m, 2H), 2.05-2.22 (m, 2H), 2.64 (m,2H), 2.98 (dd, 1H), 3.13 (t, 1H), 3.30 (m, 1H), 3.38 (t, 1H), 3.45 (m, 1H), 3.64 (m, 1H), 3.85 (m, 2H), 7.18-7.35 (m, 4H)

Manufacturing Example 4

N-[3-(2-Fluorophenyl)-2-propenyl]-1-deoxynojirimycin

Step 1: 1-(2-Phenyl)-2-propen-1-ol:

2-Fluorobenzaldehyde, 992 mg (8.00 mmols), was dissolved in 10 ml of tetrahydrofuran. The solution was cooled to -78 °C and 10 ml of 1 M vinyl magnesium bromide tetrahydrofuran solution were added dropwise thereto. After the dropwise addition the mixture was stirred for 3 hours at the same temperature. Then, the cooling bath was withdrawn followed by stirring for an hour. Under ice cooling, water was added to decompose the excess of the reactant, and the solvent was then distilled off. After 2N sulfuric acid was added to the residue to render it acidic extraction was performed with ethyl acetate. The extract was washed with water, dried and then concentrated. The residue was purified by silica gel column chromatography (ether : hexane = 1 : 10) to give 817 mg (67%) of oil.
NMR ($CDCl_3$) δ:
2.14 (s, 1H), 5.20 (d, 1H), 5.36 (d, 1H), 5.53 (br, 1H), 6.07 (ddd, 1H), 6.9-7.55 (m, 4H)

Step 2: 1-(2-Phenyl)-3-bromo-2-propene:

1-(2-Phenyl)-2-propen-1-ol, 456 mg (3.00 mmols), was reacted in a manner similar to Manufacturing Example 3, Step 4 to give the title compound.
NMR ($CDCl_3$) δ:
4,19 (d, 2H), 6.39 (dt, 1H), 7.04 (d, 1H), 7.20-7.60 (m, 4H)

Step 3: N-[3-(2-Fluorophenyl)-2-propenyl]-1-deoxynojirimycin

The title compound was synthesized in a manner similar to Manufacturing Example 1 except that 1-(2-phenyl)-3-bromo-2-propene was substituted for 1-bromo-3-phenylpropane.
NMR ($CD_3OD$) δ:
2.1-2.25 (m, 2H), 3.06 (dd, 1H), 3.14 (m, 1H), 3.24-3.35 (m, 1H), 3.39 (t, 1H), 3.50 (m, 1H), 3.71 (m, 1H), 3.94 (ABXtype, 2H), 6.45 (dt, 1H), 6.72 (d, 1H), 7.00-7.16 (m, 2H), 7.20-7.28 (m, 1H), 7.53 (dt, 1H)

Manufacturing Example 5

N-(4,4,4-Trifluorobutyl)-1-deoxynojirimycin

The title compound was synthesized in a manner similar to Manufacturing Example 1.
NMR ($CD_3OD$) δ:
1.74 (m, 2H), 1.95-2.35 (m, 4H), 2.58 (quin, 1H), 2.89 (dt, 1H), 2.98 (dd, 1H), 3.13 (t, 1H), 3.31 (t, 1H), 3.45 (ddd, 1H), 3.86 (ABXtype, 2H)

Manufacturing Example 6

N-(5,5,5-Trifluoropentyl)-1-deoxynojirimycin

The title compound was synthesized in a manner similar to Manufacturing Example 1.
NMR ($CD_3OD$) δ:
1.40-1.70 (m, 4H), 2.00-2.33 (m, 4H), 2.57 (dt, 1H), 2.85 (dt, 1H), 2.98 (dd, 1H), 3.13 (t, 1H), 3.46 (ddd, 1H), 3.85 (ABXtype, 2H)

While the invention has been described in detail and with reference to specific embodiments thereof, it is apparent to one skilled in the art that various changes and modifications can be made therein without departing from the spirit and the scope of the present invention.

## Claims

1. An antiviral composition comprising as active ingredient a N-substituted-1-deoxynojirimycin derivative represented by general formula (1):

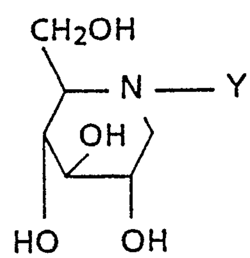

wherein Y represents

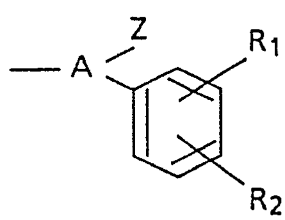

(wherein A represents a straight or branched chain hydrocarbon group having 3 to 5 carbon atoms which may or may not contain a double bond, $R_1$ and $R_2$, which may be the same or different, each represents a hydrogen atom, a lower alkyl group, a lower alkoxy group, a carboxyl group, a carbamoyl group, an alkoxycarbonyl group, a dialkylamino group or a halogen atom; Z represents a hydrogen atom, a lower alkyl group, a phenyl group or a group shown by:

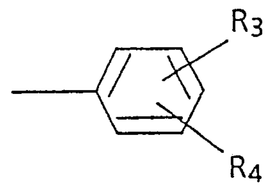

wherein $R_3$ and $R_4$, which may be the same or different, each represents a lower alkyl group, a lower alkoxy group, a carboxyl group, a carbamoyl group, an alkoxycarbonyl group, a dialkylamino group or a halogen atom) or $-(CH_2)_nCF_3$ (n represents an integer of 2 - 5).

2. A composition as claimed in Claim 1, wherein said viral infection is an infection with human retro virus.

3. A composition as claimed in Claim 1, wherein said viral infection is an infection with human immunodeficiency virus (HIV).

4. A composition as claimed in Claim 1, which is for therapy or prophylaxis of acquired immunodeficiency syndrome (AIDS).